# EUROPEAN PATENT SPECIFICATION

(11) **EP 1 962 089 B1**
(45) Date of publication and mention of the grant of the patent: **26.10.2011**
(21) Application number: 07425096.0
(22) Date of filing: 21.02.2007
(51) Int. Cl.: G01N 33/18, G01N 21/64

(54) **Expendable launchable probe for temperature and fluorescence measurements of the undersea environment**
Abwerfbare Einmalsonde für Temperatur- und Fluoreszenzmessungen in Unterseeumgebungen
Sonde à lancement extensible pour mesurer la température et la fluorescence de l'environnement sous-marin

(43) Date of publication of application: 27.08.2008
(73) Proprietor: Marcelli, Marco, 00145 Roma (IT); Di Maio, Antonia, 00154 Roma (IT); Mainardi, Umberto, 24020 Villa Di Serio BG (IT)
(72) Inventor: Marcelli, Marco, 00145 Roma (IT); Di Maio, Antonia, 00154 Roma (IT); Mainardi, Umberto, 24020 Villa Di Serio BG (IT)
(74) Representative: Zizzari, Massimo

(56) References cited:
- US-A- 4 854 728
- MARCELLI, M ET AL.: "Development of a new expendable probe for the study of pelagic ecosystems from voluntary observing ships" OCEAN SCIENCE DISCUSSIONS, 11 September 2006 (2006-09-11), pages 1515-1541, XP002460660
- MCKEE D ET AL: "An integrated submersible fluorometer/nephelometer/transmissometer: design and testing at sea" OPTICS AND LASER TECHNOLOGY, ELSEVIER SCIENCE PUBLISHERS BV., AMSTERDAM, NL, vol. 29, no. 1, February 1997 (1997-02), pages 35-39, XP004014922 ISSN: 0030-3992
- KUWANO H ET AL: "NEW MULTIMEDIA COMMUNICATION SERVICES USING SENSING SYSTEMS. DEVELOPMENT OF WATER-QUALITY SENSING NETWORK SYSTEM" NTT REVIEW, TELECOMMUNICATIONS ASSOCIATION, TOKYO, JP, vol. 9, no. 1, January 1997 (1997-01), pages 100-107, XP000643777 ISSN: 0915-2334

## Description

The present invention relates to a probe for measurement of physical, chemical or biological parameters, in aquatic or undersea environments. In case the same instrument is used for studying the undersea environment, it consists of an expendable probe, launchable from a mobile ship.
The probe has been invented and especially designed to provide measurements of fluorescence (and temperature) along the water column into the sea environment, but it can be further used in other types of environments (i.e. lakes, or artificial basins like aquaculture pools or aquariums).

The actual methodologies, for an operational monitoring of the oceans, are mainly based on *lagrangian profiling buoys* (Argo) and on *ships of opportunities.* Potentially, the Argo floats can measure physical, chemical and biological parameters. In practice, this potential capability is limited by cost and power supply. Instead, the use of *ships of opportunities* has permitted to decrease the costs of operative phase in sea research (*in situ* data acquisition) without affecting the quality and quantity of information.

Indeed, the "sea truth" is guaranteed by integration of *in situ* collected data and remote sensing data from satellites, that confirm synoptic data acquisition, but allow to sort out only the surface part of the water column; in fact data from satellites don't consider the third dimension that is deepness.

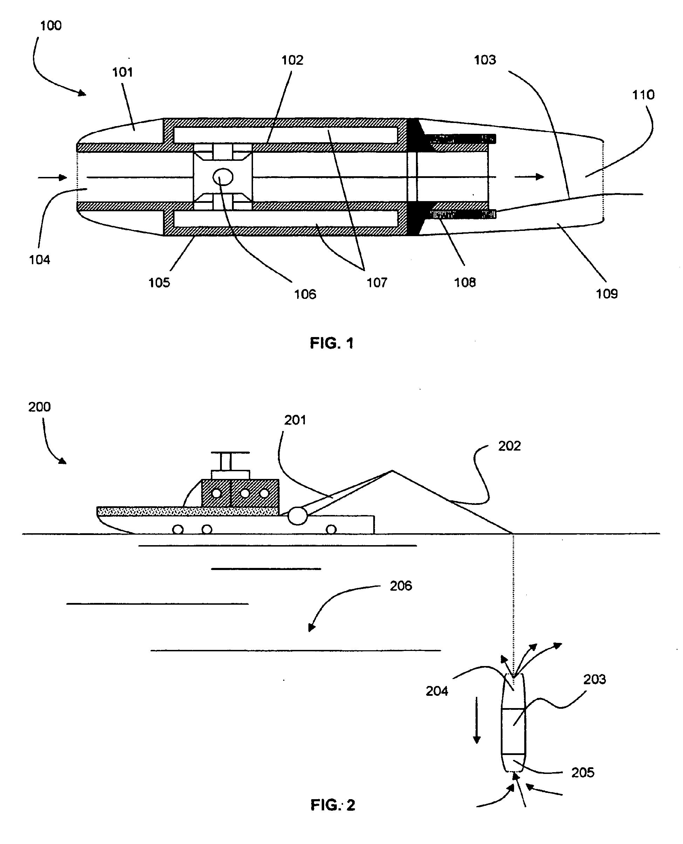

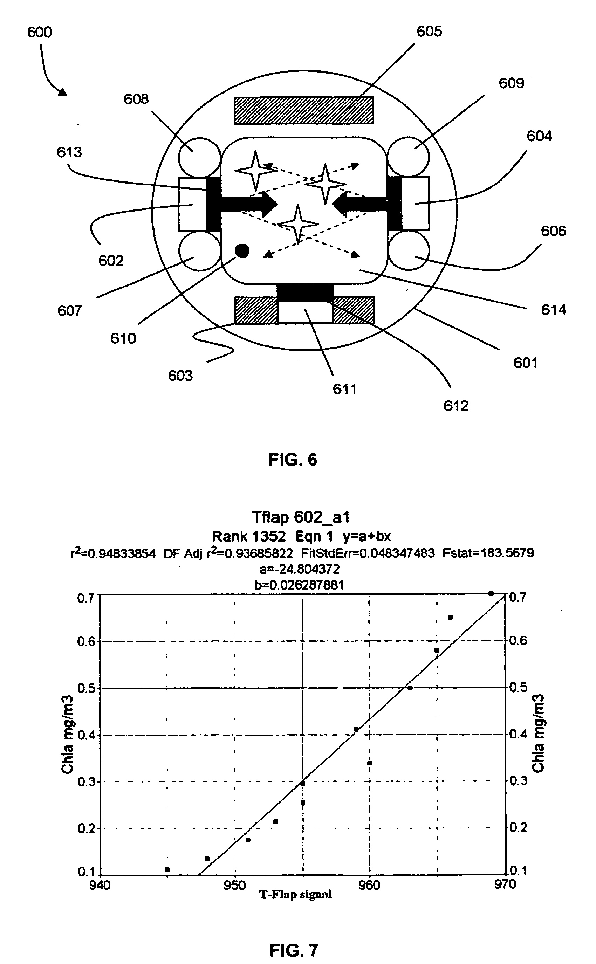

An example of observation and forecasting of physical state in *Mediterranean Sea*, based on integration of *in situ* investigations and satellites data, is active since 1999, as described in the following publication:
[1] Pinardi, N., Allen, I., Demirov, E., et al.: "The Mediterranean ocean Forecasting System, first phase of implementation (1998-2001)", Ann. Geophysicae, 21: 3-20, 2003.
   In particular, the instrument that has been used on ships of opportunities for the abovesaid purpose is the so-called XBT (*eXpendable Bathy Thermographs);* that is an expendable profiler capable to provide temperature in the surface layers of the water column.
   The XBT is not the only expendable probe actually used, because there is also the so-called XCTD (*eXpendable Conductivity Temperature Depth probe*), that is a profiler capable to measure not only temperatures, but also conductivity in the water column. However, such an instrument is not commonly used because of its expensive cost.
   Furthermore, the use of ships of opportunity has permitted to significantly decrease costs of description of "sea truth", and expendable probes are instruments particularly suitable to be used on any type of ships (that is, also from ships not specially equipped for research). However, nowadays it is much more required to obtain a significant amount of data to be integrated with satellite data in order to calibrate very precise forecast models.
   This approach has been used in a project, so-called MFS-TEP *(Mediterranean Forecasting System - Toward Environmental Prediction),* funded by European Union, through the program *Voluntary Observing System* (VOS). This program concerned the data acquisition task using exactly the XBT, in order to define an *input* for sea weather forecast models in *Mediaterranean Sea.*
   In the same project, it has been tried to answer the need of physical data integration with biological data, with particular reference to chlorophyll concentration that, in the same way like temperature, can be synoptically measured by satellite only on the surface.
   The estimation of primary production in the euphotic zone through remote sensing has been based mostly on visible-band water-leaving radiance measured with the coastal zone colour scanner. There are some robust, simple relationships for calculating integral production based on surface measurements, but they also require knowledge of photo adaptive parameters such as maximum photosynthesis which currently cannot be obtained from space, as better explained in the publication:
[2] Balch, W., Byrne, C.F.: "Factors affecting the estimate of primary production from space", Geophys. Res. J., 99, 7555-7570, 1994.
   There is no way to quantify model performance without comparing the output to *in situ* data. When asked what is needed to improve model performance, all model developers coincide in requesting more data, ideally together with ancillary data such as nutrients and community structure. Further information is in the publication:
[3] Carr, M.-E., M.A.M. Friedrichs, M. Schmeltz, et al.: "A comparison of global estimates of marine primary production from ocean colour", Deep-Sea Research, Part II, 53, 741-770, 2005.
   More than for the physical variables, the biological ones have to be observed *in situ.* Especially in the mid-low latitudes, a deep observation of the water column is needed, because of the typical distribution of phytoplankton's biomass, as specified in the following publication:
[4] Mann, K.H., Lazier, J.R.: "Dynamics of Marine Ecosystem", Blakwell Science, 1991.
   The acquisition of a huge amount of data concerning chlorophyll estimation allows to monitor the variation of sea productivity, giving further important information suitable for studies on state of ecosystem.
   This purpose can be better achieved when the estimate of chlorophyll concentration is done *in situ,* through measurement of active fluorescence, because in this case the biophysical reaction of phytoplankton's cells in their natural environment can be measured in real time; instead, the analysis in laboratory don't provide real time results and present a series of drawbacks due to manipulation of specimen.
   Actually, some fluorescence profilers exist and they answer to abovesaid characteristics (i.e. *PrimProd* developed by *Biophysical Institute of Moscow,* Russia) but they are quite expensive and can be used only from research ships.
   Fundamental characteristic of such a profiler is that it should be cheap.
   A probe capable to answer the claim of temperature and fluorescence profiler, that is cost effective and expendable at the same time, to be used in ships of opportunity is known from "Development of a new expendable probe for the study of pelagic ecosystems from voluntary observing ships", Marcelli et al., in Ocean Science Discussion, vol. 3, pp. 1515-1541, 2006. The aim of such a probe is to add biological profiling measurements, to physical and chemical ones, in order to have more extensive information on chlorophyll concentration in the sea. Chlorophyll is an index of phytoplankton's biomass, and is also the most common property that characterizes marine productivity.
   The possibility of using a continuous profiling probe, with an active fluorescence measurement, is very important in the study of phytoplankton in real time; it is the best way to follow the variability of sea productivity. In fact, because of the high time and space variability of phytoplankton, due to its capability to answer in a relatively short time to ecological variations in its environment, and because of its characteristic patched distribution, there isn't a precise quantitative estimation of the biomass present in oceans and seas. Further information is available in the publication:
[5] Lewis, M.R., Cullen, J.J., Platt, T.; "Relationship between vertical mixing and phytoplankton: similarity criteria", Mar. Ecol. Prog. Ser., 15: 141-149, 1984.
   Moreover primary producers in the sea, phytoplankton at first, contribute for the 40% of global assimilation of inorganic carbon, resulting particularly relevant in the study of global change, as specified in the publication:
[6] Falkowski, P.G.; "The ocean's invisible forest" , Scientific American 287: 38-45, 2002.
   The development of the expendable probe of this invention has followed similar concepts of the XBT (a wire conducting the signal to a computer card), but differently from that, it was developed with an electronic system which can be improved and adapted to several variables measure channels (modularity). Commercial components were used for the development of a prototype, with the aim of a low-cost probe: a glass bulb temperature resistor for the temperature measurement; blue LEDs, a photodiode and commercial selective glass filters for the fluorescence measurement. The measurement principle for the detection of the phytoplankton's biomass is the active fluorescence, which enables to have an *in vivo* chlorophyll estimation, measuring the immediate biophysical reaction of the phytoplankton inside the aquatic environment. This is a non-disruptive method which gives real time estimation and avoids the implicit errors due to the manipulation of samples.
   It is therefore specific subject of the present invention, so called *T-Flap,* a probe for measurement of physical, chemical or biological parameters, as defined in claim 1.In case said probe is used for study of the undersea environment, it consists of an expendable probe, launchable from a ship of opportunity, and said measurement cell includes, among said sensors, further devices providing measurement of fluorescence, particularly suitable to reveal real time concentrations of phytoplankton.
   Other characteristics and advantages of the invention will become apparent on examining the detailed description hereinbelow, as well as the appended drawings in which:
   Figure 1 is a longitudinal sectioned view of a launchable expendable probe;
   Figure 2 is a schematic view of the probe during the measurements of fluorescence and temperature in the undersea environment;
   Figure 3 is a schematic view of the electronic system for data acquisition and transmission, contained inside the probe;
   Figure 4 is a schematic view of the probe during calibration of instruments inside the laboratory;
   Figure 5 is a schematic view of the probe during the measurements in the undersea environment, with particularly shown the connection to a computer for processing and data presentation on a graphical board;
   Figure 6 is a front cross-sectional view of the probe showing a particular of the measurement cell;
   Figure 7 is a graphical view where, on X axis is the Chl "a" measured by T-Flap (mV) while on Y there is the Chl "a" measured by a calibrated fluorometer (PrimProd 1.08) expressed in mg/m³;
   Figure 8 is a graphical view of best fit curve for the calibration of the T-Flap fluorescence sensor: on Y is reported the Chlorophyll concentration measured by PrimProd (mg/m³) and on X is reported the signal given by the T-Flap's fluorescence sensor;
   Figure 9 is a graphical view where, on X axis there is the temperature measured by T-Flap (mV) while on Y there is the temperature measured by a probe Idronaut expressed in °C;
   Figure 10 is a graphical view of best fit curve for the calibration of the temperature sensor: on Y is reported the temperature measured by a probe OTM Falmouth (°C) and on X is reported the signal given by the T-Flap's temperature sensor;
   Figure 11 is a graphical view of a comparison between chlorophyll measurement of T-Flap (black line) and reference probe (bold black line);
   Figure 12 is a graphical view of a comparison between temperature measurement of T-Flap (black line) and reference probe (bold black line).
   It must at first underlined that in the following only some of the many conceivable embodiments will be described on the basis of the basic solution of the present invention, but it is clear that changes and/or modifications can be introduced by those skilled in the art, without departing from the relevant scope of the same invention.
   The expendable probe of the present invention can estimate the Chlorophyll "a" fluorescence and water temperature in a water column. It has a transmission system and a shape developed in order to allow its use from moving ships. The simplicity in the use of this probe and its relatively low cost were the basic requirements of the development. Other requirements were concerning the need to detect the "deep chlorophyll maximum" (DCM) with a sufficient accuracy, and temperature with an accuracy of 0.1 °C.
   The main components of the probe 100 are shown in Figure 1. Electronics and firmware 107, for sensors management and data transmission, are placed inside the probe 100. Measurements take place in an internal cell 106, where the water flows while the probe descends along the water column. The data transmission is allowed by a twin copper wire 103 wrapped on a coil 108 placed in the tail 109 of the probe 100 and in a canister.
   The probe 100 is constituted by: a central body with a measurement cell represented by a tube 102 made of a wrought aluminium alloy of the 6000 series, where the water flows in the inside; in the internal part of the tube 102 are fixed the sensors 106 which are in direct touch with the water, while on the external part of the tube are placed the batteries and the electronics 107; this central body 102 is covered by a larger tube 105 made of a wrought aluminium alloy of the 6000 series, closed by two flanges, constituting the case where are placed batteries and electronics 107; on the tail 109 is placed a coil 108 with the copper wire 103 for data transmission, the tail 109 has a hydrodynamic shape that facilitates the wire 103 unreeling; the frontal part 101 is composed by heavy material to allow a vertical fall: it is constituted by a turned zinc ogive blocked by a metallic nail.
   The electronics (Figure 6) are placed on two parallel faces of the measure cell 600. It is composed by two printed circuits 603, 605 and batteries 606, 607, 608, 609. These circuits contain the integrated electronic functions, distinct for typology:
   - circuit 1, 603, positioned at the photodiode side, has all the analogical measure functions, signals conditioning and digital conversion functions;
   - circuit 2, 605, has all the digital elaboration circuits, data transmission, LED diodes piloting functions and the power supply system.

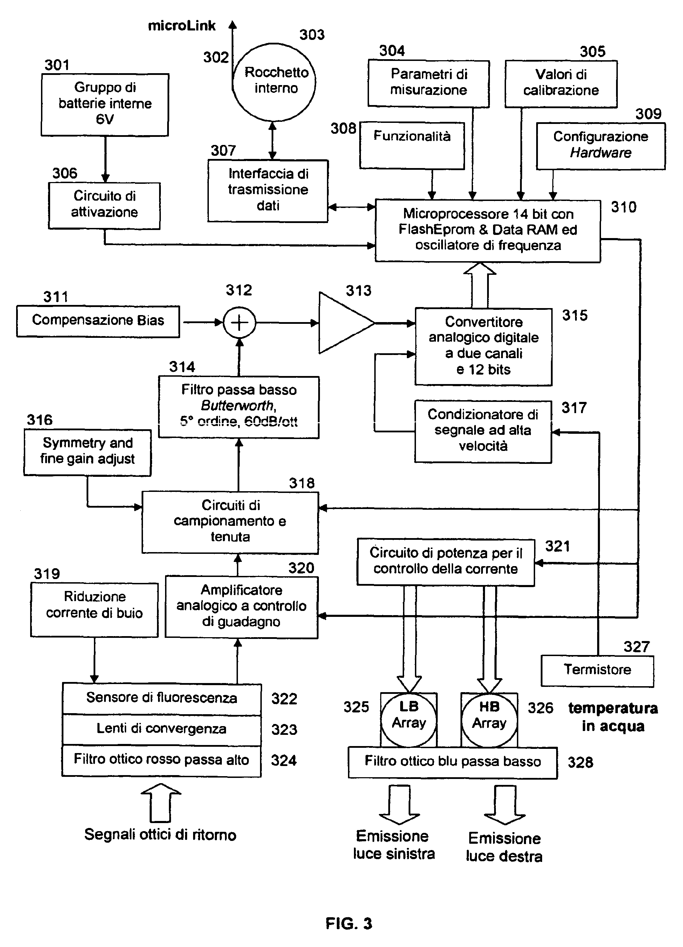

   In Figure 3 a general block view of the electronics is represented.
   The measure channel for fluorescence is composed of a silicon photosensor integrated with a current electrometric amplifier 322 and a series of optical lenses 323 to focus light signals activated in the excitation chamber. This solution enhances the signal to noise ratio and increases the speed of reaction in the optical group. The bandwidth of the optical signal is limited through a red high-pass filter 324, calibrated at the wavelength of 600 nm. On the same light detector module 322, 323, 324, an additional circuit for decreasing the black reference current 319 is placed in order to avoid the photodiode saturation in front of a very intense environmental light.
   The output measure signal from the fluorescence detector module is sent to the amplifier 320 through a decoupling circuit composed of a high-pass RC circuit whose aim is to cut the residue of the offset current. The voltage gain of amplifier 320 is directly controlled by microprocessor 310 with three ten-based steps 1-10-100 during the measurement and, in automatic mode, the computer data process defines automatically the best resolution scale and thus the sensitivity adaptation. Circuit 318 is placed in cascade to sample the synchronized clock frequency; this so called *sample and hold* circuit has the purpose to track and record a part of the analogic signal detected from the previous circuit and to keep it in memory for all along the time until next clock, where memory is made of a low leak capacitor with policarbonate dielectric. The element 318 is composed of a first decoupling stage with a high-pass RC fitter to cut off the offset from the variable gain amplifier and from an inverting amplifier where is possible to control 316 the symmetry and the fine-tuning scale factor. The second stage of element 318 has a CMOS static switch to implement the *sample* function and a memory capacitor for thr *hold* function. When the static switch is short circuited the capacitor is charged at the same voltage of the previous amplifier circuit, instead when the switch is open the same electrical charge of the previous phase is kept on the capacitor. The output signal of element 318 follows the variation of input signal only in the *sample* state, in this state all the selected leds are switched on.
   The output signal of element 318 is sent to the low-pass filter 314, that is of type *Butterworth* with 5 poles, cut-off frequency of 5 Hertz, and high fading depending on frequency equal to 60 Db/oct. The filter circuit amplifies the signal with a 2.54 coefficient, and the general measurement chain gain can reach 2 V/µV or 1 V/nA. The purpose of the filter is to average the measure in the short term and to balance the signal flotation respect to interference noises that are generated by flow turbolence as typical dynamic phoenomenon; the resulting background noise is less than ± 1 digit. In order to complete the signal processing, an adder stage 312, 313, is placed and receives a bias adjust signal from 311 so that the thermal effect of bias currents in the filter stages is decreased. Thus, the output signal is function of the pulse and reactive light that is caught in the optical chamber over 600 nm wavelength.
   The water temperature is detected by thermistor 327 and the related signal is converted by signal conditioner 317 that is composed of a *Wheatstone bridge* circuit and a differential amplifier. Temperature is measured as a resistor value:
   inside the *Wheatstone bridge* the electrical signal is balanced at -10°C, and the output signal from amplifier depends on thermal difference with reference to 0 °C. At 25 °C of positive temperature corresponds a 1 V signal beyond the balance value. For this type of signal are not required further conditioning, so the output signal of the integrated circuit is decoupled with a low-pass RC filter and
   sent to A/D converter 315. The thermal mass of the temperature sensor is extremely small and less than one millimeter plus 0.25 mm of insulating glass; this mass guarantees a very quick response to temperature variations in order of 0.05 s/°C.
   The signals of fluorescence and temperature reach a unique A/D converter 315 having two analogic input channels 0-5 V. Conversion is made by successive approximations, the conversion coefficient (digits/mV) is calibrated at the reference voltage, and one conversion cycle is executed in 50 µs per channel with a resolution of 12 bits. The high conversion rate, 20.000 data per second, allows a quite continuous digital processing on a relatively huge quantity of data. The time delay between the two channels is kept lower than one millisecond, so that is possible to obtain a result with minimum displacement and, however, highly correlated.
   The two groups 325 and 326, LB and HB, of light emission for cell excitation, are composed of three LED diodes each. They are of high efficiency and light type. Each single LED diode, in a single module, radiates light with different wavelengths, 430-450-470 nm. Each module is driven by a power circuit 321, that controls the current in the three diodes and modulates it at the measure frequence, in the range of nearly 250-1000 Hz. The *boosters* are controlled by a logic port that receives a command by microprocessor 310 and the modulation frequency by oscillator 310. It is not strictly required a big precision and stability of modulation frequency, it can have a variation of ± 15% without leading to significant measurement errors, decrease of sensitivity or lack of efficiency in LED diodes. Furthermore, the two groups are equipped of band-pass filter 328 working in the blue region nearly between 410-470 nm.
   A low consumption microprocessor 310 is embedded as a control and processing unit, model *PIC17F876* CMOS *14 bits Microchip,* instruction cycle: 200 ns, program flah memory 8 kbytes, EEPROM memory 256 bytes, RAM memory 356 bytes, 2 PWM outputs, serial and catch functions. In this system a 20 MHz quartz oscillator and a second 250 KHz RC oscillator (as modulator) have been integrated. The microprocessor 310 controls: the communication with the data terminal, the measure scales, the data acquisition through the A/D converter 315, the data processing, the serial data transmission through interface 307, the clock cycles, the functional logic of the start-up system 306, the *"start on sea"* and auto *"shunt off".*
   The data process consists of providing a first average, or integral-similar, of the sampled data within a complete modulation phase, 2+2 ms, in order to integrate the typical measurement noise. Then, a following procedure collects data from the first average on a stack, cuts off the minimum and maximum values, and makes another average of the remaining ones. The stack is floatant, thus at each average measure cycle the oldest value is deleted and the latest one is saved. The size of stack can be defined by a interactive menu. The data, equally processed on both channels, are then formatted and transmitted to computer on board through *microlink* 302.
   The T-flap is equipped of a *firmware* program able to communicate with a user, through a remote terminal, in order to set up the instrument with the best measure conditions, allowing the definition of a series of parameters concerning: device 308 functionalities, *hardware* 309 configuration, measurement 304 and data processing mode, calibration values 305 and sensitivity given by calibration process.
   In order to execute the above operations it is required a data terminal or a *personal computer* (PC) with a terminal program, like in example *Windows® HyperTerminal.* It is possible to use a serial converter RS485/232 that is connected on the Rx485 side to the *microlink* wires, and on the RS232 side to the serial port of PC. The communication rate is 9600 baud, no parity check, one bit stop, and no flow control.
   The parameters 304 are related to: definition of gain coefficient for fluorescence measure channel; definition of number of samples + 2 values in the stack concerning floating average calculation (AVG) (this number is between 1-9); definition of data acquisition and communication speed; definition of latent time between two continuous transmission strings (value between 0-9 corresponds approximately to 0-900 ms).
   With reference to the calibration 305, the measure system is switched on ad set up for the calibration process, data transmission is slower, the mathematical process is set to maximum precision, data strings are linked to functional information.
   With reference to functions 308, the device's functionalities require that user executes the calibrations for measurement of fluorescence and temperature, directly in a laboratory. After that, the calibrated device is switched to the function as *monitor* or launch preparation. This latter function should be activated immediately before the launch in the sea.
   The configurations 309 include the control of LED groups, 321, 325, 326, wherein: both groups are off; only the left group or the right group is on and modulated; both groups are on and modulated. Furthermore, the same configurations 309 include: the *standard* operative function; the definition of the serial number (possible only on test phase); reset of the previous definitions and set up with the *default* values; activation of the *shutdown* function; activation and control of the automatic switch on function at the moment of impact on sea.
   The data transmission interface 307 converts serial information TTL to a proper signal level for long distance transmission up to 1500 m, through a very thin enamelled copper wire and wrapped on an internal coil 303. This interface can provide data transmission either with the two-wires technique RS485 modified or single-wire with the modem V.23 1200/75 FSK technique. The umbilical cable 302 is characterized by intrinsic properties like: conductor cross-section, thickness of insulating material, distance between conductors, copper resistivity, the conductors total length. Furthermore, it comprises extrinsic properties, like: the wrapped length respect to unreeled length, electrical impedance that is continuously variable during the descent run, variation of resistance dependant on temperature, stray capacitance to water, etc.
   These parameters continuously change during the T-flap's descent run, decreasing from time to time the inductance value but increasing the stray capacitance, while the contribution of temperature has a marginal effect.
   Experimental characteristics of elements 302 and 303 are the following ones:
   - total resistance (measured on both conductors): 11,26 ohm/m @ 21 °C;
   - coupled capacitance (between conductors) : 170 pF/m @ 21°C;
   - stray capacitance or leaking capacitance : 1417 pF/m @ 50 mS;
   - specific inductance (wire wrapped on two coils): 2.01 H on 1500 m wrapped;
   - impedance Z measured at 1000 Hz: 13.63 Kohm on 1500 m @ 50 mS.
   The power supply 301 of the above device is provided by 4 alkaline batteries of AAA type, watertight, able to supply the electronic system of required energy for a time limit longer than necessary for launch, up to 100-150 minutes with alkaline *Duracell* batteries.
   The activation 306 of the device happens on contact with sea water. Activation means that the device is working as programmed and ready to be launched. In order to provide the calibration process, or testing the equipment, it can be supplied by an external battery, but this is no more possible after that T-flap is sealed and prepared for launch. In practice, the contact of internal batteries with sea water electrically connects the electronic circuits, and all the system switches on and prepares for expected task, so that batteries are connected fo undefinite time up to the final discharge of energy.
   With reference again to the probe, the optimal shape-structure is 8 cm diameter for a 35.9 cm length. These dimensions assure a good weight distribution and, as a consequence, a good vertical lowering of the probe.

The zinc ogive can be more or less heavy bringing to a total weight in water from 1.50 Kg to 2.15 Kg. The fluorometer is composed by:
- a source of light 602, 604 in the wavelengths of 430nm, 450nm, 470nm (blue LEDs);
- an optical filter 613, 614 which selects the blue wavelength from 430 to 480 nm;
- an amplified 611 semiconductor element (with electronics integrated) as sensitive receptor;
- an optical filter 612 which starts to select the red light from 600 nm reaching the maximum of transparency at 680 nm.

The temperature measurement is effectuated through a glass bulb micro sensor 610 which comes out from the measure cell for 10 mm, the sensitive part is composed by resistive sensor inside a spherical glass bulb with the diameter of 1.5 mm. This sensor has a high sensitivity to temperature variations (0.01 °C) and to the dynamic variations (0.05 ms). Depth was measured through a submersible pressure transducer (*Keller*). The pressure transducer was inserted only on the first two prototypes used in the development phase.

Data transmission (Figure 2) is digital and is allowed by a twin copper wire (*microlink*) 202 wrapped on two separate coils: one of these 204 is one-block with said device 100 and, positioned in the tail, rotates together with the whole instrument, while the other one is fixed in the canister launcher 201; through the copper wire 202 the probe is connected to a *personal computer* (PC) on board.

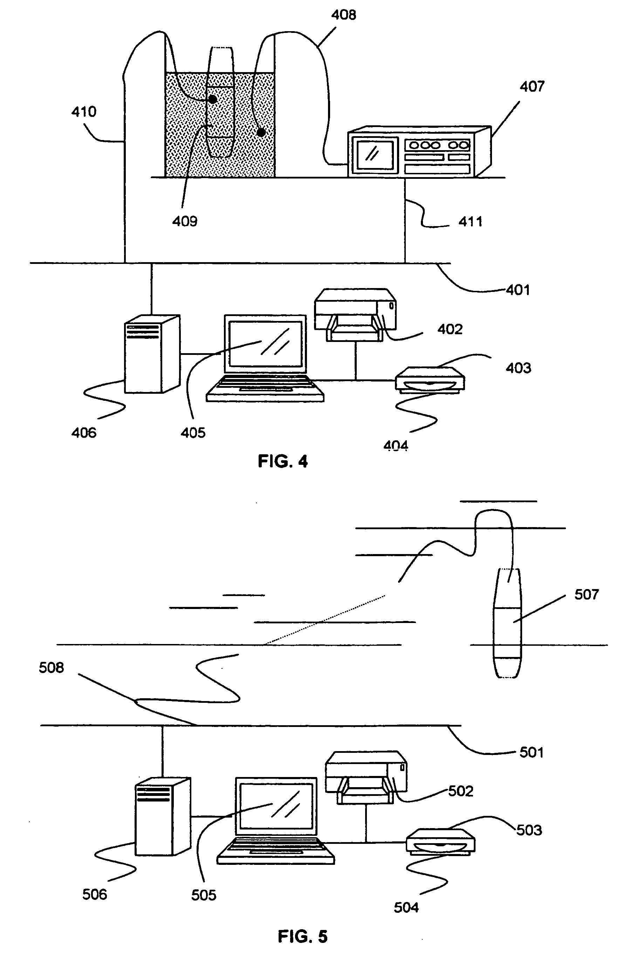

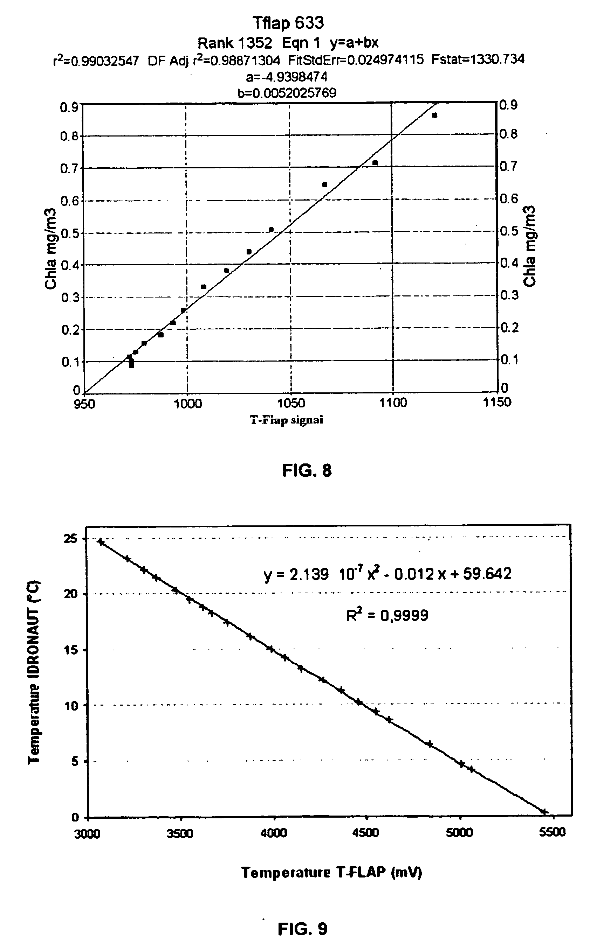

Data acquisition doesn't need specific software, it is sufficient to have a PC terminal as *Windows*® *HyperTerminal* and follow the information transmitted by the connected device. The interactive menu allows different work activities including programming and controlling.
The mobile coil, inside the probe (Figure 1) 100, has to contain a wire 103 with a length as the maximum depth it could reach plus a tolerance length: the pre-serie has 700 meters wire.
The main application for which the expendable probe was designed is the profiling measure of fluorescence and temperature along a water column. Therefore, being subjected to a progressive hydrostatic pressure, the functional limits of the instrument, concerning to the implosion of the materials, stand by 500 dBar, expecting this to be the highest pressure we need to reach.
A second limit is the capability of the coils to host a longer or thicker couple of conductors: for each one it was prevented at the most a double length with the same diameter, or a bigger diameter with the present length.
The development of the probe has followed two main phases. The first phase of the experimental activities for the fluorometer assembly has been directed to an accurate evaluation of any element which was able to full fit the characteristics of the instrument's components (LEDs, diodes, filters), with particular attention to low cost components. The experimental study had the aim to find the best combination of the selected elements.
The spectral characteristics of different LEDs lights were analysed to detect the eventual presence of emissions in the red band, which would interfere with the chlorophyll measure. Afterward it was verified the transmittance of the filters and it was analysed the spectral emission of the LED with blue and red filters.
The analysis of LEDs spectra and filters transmittance was done with an EPP2000C UV+VIS Spectrometer (Wavelength Range 200-850 nm; resolution 0.75 nm). Once found the best transmittances, selected LEDs, combined with the selected filters, have been tested in the *Laboratory of Experimental Oceanology and Marine Ecology* in Civitavecchia - Italy.
LEDs, filters and photodiodes were assembled in a not submersible measurement cell. By using an oscilloscope, the photodiode output signal was measured to detect the 'parasite' light (deduced from the noise signal).
Different filters were analysed such as acetylene filters used for a first prototype (LEEfilters) and glass photographic coloured ones. Once verified that there was no appreciable interference from glass on light emission, we had reason to utilize it as support for the filters. A final choice of the filters was operated in favour of photographic ones, in order to have retrievable and cheap components.
When the definitive geometry of the measure cell was defined, fluorescence measurements were carried (Figure 4) in order to study the sensor's output signal and define its amplification. After each modification at the fluorometer, the quality of the output data was tested with different concentrations of *in vivo Chlorella sp.* cultures, which were analysed otherwise with spectrophotometer method.
In the second phase, on the basis of this preliminary experimentation, different prototypes were assembled and tested. At first two retrievable prototypes were assembled, which enabled to carry out *in situ* tests (Figure 5), analysing the output signals and bringing the required modifications (on the gain and on the optics). The first two retrievable prototypes have the measurement cell a little larger and are geometrically different from the definitive probe. Moreover these are provided with a pressure transducer that the expendable ones do not have, because of the need of a low cost instrument.
The study carried with the retrievable prototypes brougth to the assemblage of the first 30 pre serie expendable probes.
Different dilutions of a concentrate *Chlorella sp.* solution measured with a calibrated fluorometer (PrimProd 1.08) were used for the static calibration of the new probe. PrimProd is a very sensible fluorometer, based on a photomultiplier sensible element developed by the *Institute of Biophysics of Moscow,* as described in:
[7] Antal, T.K et al.; Measurements of phytoplankton photosynthesis rate using a pump and probe fluorometer, Oceanologia 43, 291-313, 2001.
   The spectrophotometer analysis of the samples employed for the calibration of the fluorometer was made following the method reported by:
[8] Lazzara et. al.; Pigmenti Clorofilliani. Nova Thalassia, vol. II, 207-223, 1990.
   In Figure 7 is shown the relation between fluorescence measurements of the first prototype (number 602) and of the reference probe (PrimProd 1.08).
   The Chlorophyll "a" concentration range, used for the calibration of the fluorescence measurement (which regression curves are shown in Figure 7 and Figure 8) goes from 0.08 mg/m³ - 0.1 to 0.9 mg/m³: the reason for choosing this range is the grater difficulty of the tested probe to detect the small Chlorophyll concentrations, while for the higher ones (the tests were leaded reaching at most 2 mg/ m³) there is a good linear relation between the calibrated probe's signal and the real Chlorophyll "a" concentration.
   Moreover the Tyrrhenian sea, where the probe was tested, is an oligotrophic system, where the mean biomass corresponds to a range of Chlorophyll "a" concentrations between 0.06 and 0.6 mg/ m³, as specified in:
[9] Marcelli et. al., Deep Chlorophyll maximum distribution in the central Tyrrhenian Sea described by a towed ondulating vehicle, Chemistry and Ecology, vol 21: 351-367, 2005.
   The first prototype, the retrievable one, has the measurement cell a little larger and geometrically different from the definitive probe, that's why in the calibration of the two types of probes (the retrievable and the expendable) there are differences in the regression coefficients.
   In order to acquire data simultaneously it was built a water flux circuit where the probes were inserted together with a reference fluorometer (PrimProd 1.11): the water flowed thanks to a bulk insulated tube system connected to a circulation/feeder pump (0.7 bar, 5.7 l/min) which kept a constant flow.
   In this way it has been possible to effectuate a dynamic calibration, which is necessary to test an instrument that descends in the water column with a certain speed, letting the water flow inside of it.
   In Figure 8 is represented the best fit curve for the calibration of the fluorescence sensor of one of the *pre serie* expendable probes (number 633).
   The reference probe, PrimProd 1.11, is a submersible PC-controlled fluorometer (accuracy: 0.1 µg/l) which uses the double flash "pump and probe" technique for measuring the constant and variable *in vivo* chlorophyll fluorescence, to estimate phytoplankton's abundance and the quantum efficiency of phytoplankton's photosynthesis, where a photomultiplier is the sensitive element. See details in:
[10] Piermattei et al., Analysis of mesoscale productivity processes in the Adriatic sea: comparison between data acquired by SARAGO, a towed ondulating vehicle, and by CTD casts, Chemistry and Ecology, vol 22, supp.1: S275 - S292, 2006.
   With reference, instead, to the temperature measurement, the response of the temperature resistor is converted in a tension signal value. To find out the conversion law, which allows transforming the mV signal value in °C, the prototype was immerged in temperature controlled water and the degree temperature value has been obtained by a reference temperature sensor. The temperature measurement inside the tube was done by means of an IDRONAUT 316, multiparametric probe, which has got an accuracy of 0.003°C.
   For each of the 22 temperature values, correspondent voltage values were extracted from sensors and a regression analysis carried out. (Figure 9 shows the calibration curve for the retrievable prototype, n.602).
   The temperature sensor was dynamically calibrated using the same flow system employed for the fluorescence calibration: this time the reference probe was a Falmouth OTM with Platinum Resistance Thermometer which has an accuracy of 0.003 °C.

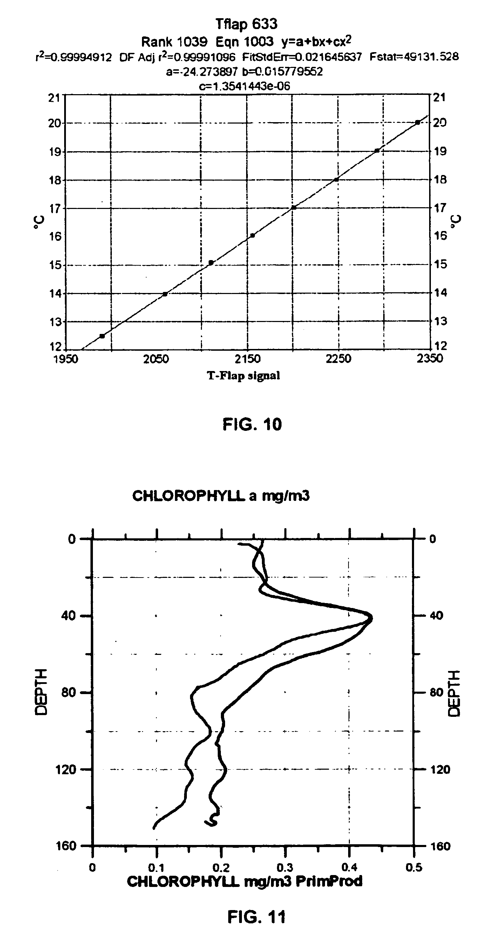

   In Figure 10 is represented the best fit curve for the calibration of the temperature sensor of one of the *pre serie* expendable probes (number 633). Field tests with the first retrievable prototypes were carried out on November 2005, during MedGOOS11 Cruise in the southern Tyrrhenian Sea.
   The reference probes utilized for fluorescence and temperature measurements were: a Seatec Fluorometer and Seabird 911.

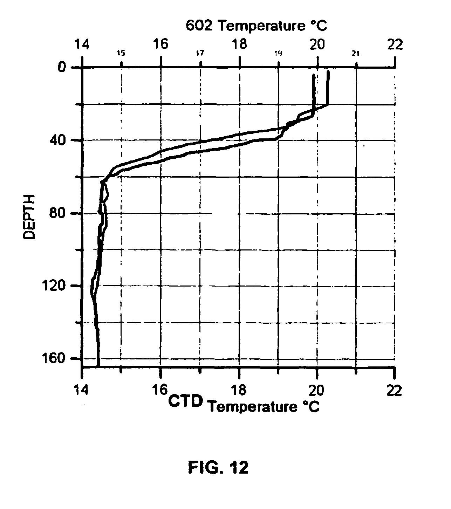

   In Figures 11 and 12, are shown a fluorescence and a temperature profile of a retrievable prototype (n.602) compared to the reference measurements.
   The water in this period is still warm in the upper layer; it is appreciable the resolution of the thermocline and the fluorescence measure can successfully describe Chlorophyll "a" distribution in the water column. Similar results were obtained in all the field tests where the "Deep Chlorophyll Maximum" (DCM) is clearly discerned.
   It is appreciable a discrepancy between PrimProd's 1.08, and tested probe's profiles.
   In both graphs (Figure 11 and Figure 12) the chlorophyll and temperature profiles of the prototype don't follow the calibrated probe ones.
   The discrepancy that we can see in the first meters is due to a saturation effect on the photodiode of the prototype, which disappears after a few seconds.
   Under the DCM it becomes relevant the less sensitivity of the expendable prototype respect to the reference fluorometer.
   Moreover the high falling speed in the first meters enhances the saturation phenomena which, even if takes place in the superficial layer, affects the signal until nearly 20 meters of depth.
   As shown in Figure 11 and Figure 12 the expendable probe's profiles seem to be shifted respect to the reference ones: this must be due to the falling rate equation employed to find out the signals corresponding depth.
   The fall rate equation adopted to estimate the probe's falling speed was obtained from the studies referred to XBT :
[11] Reseghetti et al., Improved quality check procedures of XBT profiles in MFS-VOS, Ocean Science Discussions, Vol. 3, pp 1441-1480, 2006.
   The equation adopted for the XBT is specific for a probe launched from an high of two meters while the prototype of the present invention was left at the water surface: for XBT the falling rate decreases along the water column before reaching a constant speed, while for the prototype of the present invention (in this case) the velocity becomes greater before reaching a constant speed. This is due to different heights from the sea surface, from where the probes are launched.
   The development of a new expendable probe for the measurement of temperature and fluorescence of Chlorophyll "a" has been based on system components having a low cost, available in commerce and purchasable by stocks in order to allow a massive production, as required by an expendable probe. Temperature measurements have reached the accuracy of 0.01 °C. The fluorescence measurement, even if the probe was tested in an oligotrophic sea, reaches an accuracy which satisfies the aim of a clear detection of the "deep chlorophyll maximum" (DCM): this ensures the description of phytoplankton's biomass in most of the seas which are more productive than the Tyrrhenian. Of course, the present invention is not limited to the embodiment described previously by way of example, it extends to other variants. Thus, additional devices for physical, chemical or biological measurements may be integrated in the above described probe. Different uses, in different aquatic environments, like rivers, lakes, aquariums, swimming pools are part of this inventions. Furthermore, it is clear that changes and/or modifications can be introduced by those skilled in the art, without departing from the relevant scope of the same invention, as specified in the enclosed claims and illustrated in the enclosed drawings.

## Claims

1. Probe (100) for the measurement of physical, chemical or biological parameters in water, comprising:
- a central body (102) made of a tube made of a wrought aluminium alloy of the 6000 series, where water flows inside; in the internal part of the tube (102) a measurement cell (106) is fixed, where sensors are placed in direct touch with water and on an external part of the tube, batteries and electronics (107) are placed;
- said central body (102) and said external part with batteries and electronics (107) are covered by a larger tube made of a wrought aluminium alloy of the 6000 series, (105), closed by two flanges, that constitutes the case where batteries and electronics (107) are placed;
- said measurement cell (106) has characteristics of modularity, and includes a series of housings, wherein respective sensors are placed for measurement of physical, chemical or biological parameters; said sensors are directly connected to said batteries and controlled by said electronics (107);
- on the frontal part (101), an element composed of a heavy material to allow a vertical fall thereof, constituted of a metallic ogive blocked by an elastic metallic ring,
so that the same probe (100) is used for the study of the undersea environment, consisting of an expendable probe, launchable from a ship of opportunities;
and wherein: said electronics (107) are placed on two parallel faces of said measurement cell (600), being composed of two printed circuit boards (603, 605) that contain the integrated electronics functions, separated according to the following typology:
- a first printed circuit board (603) positioned at a photodiode side, which first printed circuit board performs all analogue measurement functions, signal conditioning and digital conversion functions;
- a second printed circuit board (605) including all digital processing circuits, data transmission, LED diodes controlling functions and a power supply system;
**characterized in**
- said electronics including a microprocessor (310) and performing said analogue measurement functions by including a silicon photosensor (322) having an integrated amplifier, with means for decreasing the black reference current (319) performed at the first circuit stage; followed by a digital gain control amplifier (320), followed by a sample-and-hold circuit (318), followed by low-pass, 5th-order BUTTERWORTH filter;
and including a single block composed of a red highpass filter (324), calibrated at the wavelength of 600 nm, and a series of optical lenses (323) to focus light signals activated in the excitation chamber of said silicon photosensor;
- on a tail (109) a coil (108) with a copper single-wire (103) for data transmission, wrapped on the same internal coil (108), in a tail (109) having a hydrodynamic shape to facilitate the unreeling of wire (103);
- on a tail (109) a coil (108) with a copper single-wire (103) for data transmission, wrapped on the same internal coil (108), in a tail (109) having a hydrodynamic shape to facilitate the unreeling of wire (103);

2. Probe (100) for the measurement of physical, chemical or biological parameters in water, according to Claim 1, further including further devices providing measurement of fluorescence composed of:
- a source of light (602, 604) in the wavelengths of 430nm, 450nm, 470nm (blue LEDs);
- an optical filter (613, 614) which selects the blue wavelength from 410 to 470 nm;
- an amplifier semiconductor element (611), with integrated electronics, as a sensitive receptor;
- an optical filter (612) which starts selecting red light from 600 nm reaching the maximum of its transparency at 680 nm.

3. Probe (100) for the measurement of physical, chemical or biological parameters in water, according to Claim 1, **characterized in that** said electronics (107) has characteristics of modularity, consistently with said measurement cell (106), including a series of connections and control circuits, respectively to said sensors for the measurement of physical, chemical or biological parameters in water.

## Patentansprüche

1. Die abwerfbare Einmalsonde (100) für Temperatur- und Fluoreszenzmessungen in Unterseeumgebungen umfasst:
- einen Zentralkörper (102) bestehend aus einem Rohr mit einer Aluminiumknetlegierung der Serie 6000, in dem Wasser fließt; im inneren Teil des Rohres (102) ist eine Messzelle befestigt, (106) an der Sensoren in direkten Kontakt mit dem Wasser verteilt sind, während am äußeren Bereich des Rohres die Batterien und die Elektronik angebracht sind (107);
- besagten Zentralkörper (102) und besagten äußeren Bereich mit Batterien und Elektronik (107), die mit einem Rohr aus beständigem Material verdeckt sind. Dieses Rohr (105) ist größer als die Aluminiumknetlegierung der Serie 6000 und an seinen beiden äußeren Enden geschlossen und bildet das Gehäuse, an dem die Batterien und die Elektronik (107) angebracht sind;
- besagte Messzelle (106), die Modularitätseigenschaften hat und eine Reihe an Gehäusen umfasste, in denen die entsprechenden Sensoren für die Messung der entsprechenden Temperaturen und Fluoreszenzen eingefügt sind; besagte Sensoren sind direkt mit den besagten Batterien verbunden und werden durch die besagte Elektronik (107) kontrolliert;
- im vorderen Teil (101) ein Element aus schwerem Material, das einen vertikalen Fall ermöglicht, welchen eine Metallspitze durch einen elastischen Ring blockiert,
so dass die gleiche Sonde (100) für das Studium der Unterseeumgebungen verwendet werden kann und die aus einer "Einmalsonde", abwerfbar von einem möglichen Schiff besteht;
und in welchem: die besagte Elektronik (107) auf zwei parallelen Seiten der Messzelle (600) angeordnet ist und aus zwei Leiterplatten besteht (603, 605), die alle integrierten elektronischen Funktionen, die für die Typologie bestimmt sind, umsetzen:
- eine erste Leiterplatte (603), die sich auf der Seite der Fotodiode befindet, leitet alle analogen Messfunktionen, Verarbeitung des Signals und Umwandlung in ein digitales Format;
- eine zweite Leiterplatte (605) umfasst die digitale Signalverarbeitung, Übertragung der Daten, LED- Dioden, die die Funktionen und das äußere Versorgungssystem verwalten,
das **dadurch gekennzeichnet ist, dass**:
- besagte Elektronik einen Mikroprozessor (310) umfasst, der die obengenannten Messfunktionen mittels eines Lichtsensors aus Silizium (322) verwaltet. Dieser Lichtsensor hat einen integrierten Verstärker mit einem automatischen Ausgleich des Dunkelstroms (319), der während der ersten Stufe des Kreislaufs erfolgt; gefolgt von einer Spannungsverstärkung des Verstärkers (320), gefolgt von einer Abtast-und Halteschaltung (318), gefolgt von einem *Butterworth-* Tiefpassfilter der 5. Ordnung;
- und umfasst einen einzelnen Block, bestehend aus einem roten Hochpass- Filter (324), kalibriert auf eine Wellenlänge von 600 nm und aus einer Reihe von optischen Linsen (323) für die Fokussierung der Lichtsignale, die in der Erregungskammer des genannten Lichtsensors aus Silizium sind;
- und umfasst einen Hauptstromkreis (321), der den Strom in den genannten LED-Dioden kontrolliert und ihn gemäß Messfrequenz, die im Intervall zwischen 250 und 1000 HZ variiert, unter Kontrolle des besagten Mikroprozessors (310) moduliert, und
- auf dem Heck (109) eine Spule (108) mit einem eindrahtigen Kupferkabel (103) für die Übertragung der Daten. Dieser Draht hat eine hydrodynamische Form, die die Abwicklung des Kabels (103) erleichtert.

2. Die Sonde (100) für die Temperatur- und Fluoreszenzmessungen der Unterseeumgebungen nach den Patentansprüchen umfasst ein weiteres Gerät für die Fluoreszenzmessungen, das wie folgt zusammengesetzt ist:
- eine Lichtquelle (602, 604) mit einer Wellenlänge von 430nm, 450 nm, 470 nm (blaues LED);
- ein optischer Filter (613, 614), der die Wellenlänge von 430 bis 480 nm auswählt;
- ein Halbleiterverstärkerelement (611) mit integrierter Elektronik wie sensible Rezeptoren;
- einem optischen Filter (612), der beginnt, das rote Licht von 600 nm auszuwählen und eine maximale Transparenz von 680 nm erreicht.

3. Die Sonde (100) für die Temperatur- und Fluoreszenzmessungen der Unterseeumgebungen nach den Patentansprüchen, die **dadurch gekennzeichnet ist, dass** die besagte Elektronik in Übereinstimmung mit besagter Messzelle Modularitätseigenschaften hat (106) und eine Reihe an Verbindungen und Überwachungskreisen, entsprechend der obengenannten Sensoren für die Temperatur- und Fluoreszenzmessungen der Unterseeumgebungen umfasst.

## Revendications

1. Sonde (100) à lancement extensible pour mesurer la température et la fluorescence de l'environnement sous-marin, comprenant:
- un bloc central (102) composé d'un tube d'un alliage d'aluminium forgé série 6000, où l'eau circule à l'intérieur; dans la partie interne du tube (102) est fixée une cellule de mesure (106) dans laquelle sont placés des capteurs en contact direct avec l'eau, tandis qu'à l'extérieur du tube sont installées les batteries et l'électronique (107);
- ledit bloc central (102) et ladite partie externe avec les batteries et l'électronique (107) sont recouverts d'un tube en matière inaltérable (105) plus grand, d'un alliage d'aluminium forgé série 6000, fermé aux deux extrémités, qui constitue l'enveloppe où sont installées les batteries et l'électronique (107);
- ladite cellule de mesure (106) possède des caractéristiques de modularité, et comporte une série de logements à l'intérieur desquels sont intégrés des capteurs respectifs pour mesurer la température et la fluorescence; lesdits capteurs sont directement connectés auxdites batteries et contrôlés par ladite électronique (107);
- à l'avant (101), un élément composé de matériel lourd permettant une chute verticale, comme une ogive métallique bloquée par un anneau élastique,
de telle sorte que cette même sonde (100) soit utilisée pour étudier l'environnement sous-marin, et consistant en une sonde "jetable" qui peut être lancée depuis un navire d'opportunité;
et où: ladite électronique (107) est placée sur deux côtés parallèles de la cellule de mesure (600), et est composée de deux circuits imprimés (603, 605) qui implémentent toutes les fonctions électroniques intégrées, distinctes selon le type:
- un premier circuit imprimé (603) situé du côté de la photodiode, gère toutes les fonctions de mesure analogique, traitement du signal et conversion en format numérique;
- un deuxième circuit imprimé (605) inclut tous les circuits de traitement numérique, transmission de données, diodes LED qui gèrent les fonctions et le système d'alimentation externe,
**caractérisée en ce que**:
- ladite électronique comprend un microprocesseur (310) qui gère lesdites fonctions de mesure à travers un photosenseur au silicium (322) ayant un amplificateur intégré, avec une compensation automatique du courant d'obscurité (319) effectuée dans le circuit du premier étage; suivie d'un gain en tension de l'amplificateur (320), suivi d'un circuit d'échantillonnage et de maintien (318), suivi d'un filtre *Butterworth* passe-bas du 5^{ème} ordre;
- et comprenant un bloc unique composé d'un filtre rouge (324) passe-haut, calibré à une longueur d'onde de 600 nm, et d'une série de lentilles optiques (323) pour la focalisation des signaux lumineux présents dans la cellule d'excitation dudit photosenseur au silicium;
- et comprenant un circuit de puissance (321), qui contrôle le courant dans lesdites diodes LED et le module à la fréquence de mesure, qui varie dans une gamme comprise entre 250 et 1000 Hz environ, sous le contrôle dudit microprocesseur (310), et
- à l'arrière (109), une bobine (108) avec un câble en cuivre monofilaire (103), pour la transmission de données, enroulé sur cette même bobine (108), ladite partie arrière (109) ayant une forme hydrodynamique qui facilite le déroulement du câble (103).

2. Sonde (100) à lancement extensible pour mesurer la température et la fluorescence de l'environnement sous-marin, selon la revendication 1, comprenant en outre des dispositifs pour mesurer la fluorescence, qui sont composés:
- d'une source de lumière (602, 604) avec des longueurs d'onde de 430nm, 450 nm, 470 nm (LED bleu);
- d'un filtre optique (613, 614) qui sélectionne la longueur d'onde de 430 à 480 nm;
- d'un élément semi-conducteur amplificateur (611), avec électronique intégrée, comme récepteur sensible;
- d'un filtre optique (612) qui commence à sélectionner la lumière rouge de 600 nm, en atteignant le maximum de transparence à 680 nm.

3. Sonde (100) à lancement extensible pour mesurer la température et la fluorescence de l'environnement sous-marin, selon la revendication 1, **caractérisée en ce que** ladite électronique possède des caractéristiques de modularité, conformément à ladite cellule de mesure (106), et comprend une série de connexions et de circuits de commande, respectivement auxdits capteurs pour mesurer la température et la fluorescence de l'environnement sous-marin.
